# EUROPEAN PATENT SPECIFICATION

(11) **EP 4 329 603 B1**
(45) Date of publication and mention of the grant of the patent: **17.09.2025**
(21) Application number: 22923529.6
(22) Date of filing: 14.12.2022
(51) Int. Cl.: G01R 33/28, G06N 20/00, A61B 5/055

(54) **COIL FAULT DETECTION METHODS AND SYSTEMS**
VERFAHREN UND SYSTEME ZUR ERKENNUNG VON SPULENFEHLERN
PROCÉDÉS ET SYSTÈMES DE DÉTECTION DE DÉFAUTS DE BOBINE

(30) Priority: 26.01.2022 CN 202210093430
(43) Date of publication of application: 06.03.2024
(73) Proprietor: Shanghai United Imaging Healthcare Co., Ltd., Shanghai 201807 (CN)
(72) Inventor: SUN, Tao, Shanghai 201807 (CN); TONG, Li, Shanghai 201807 (CN); LU, Hai, Shanghai 201807 (CN); HU, Kairui, Shanghai 201807 (CN)
(74) Representative: Wang, Bo
(86) International application number: PCT/CN2022/138994
(87) International publication number: WO 2023/142742

(56) References cited:
- WO-A1-2021/064194
- CN-A- 103 376 435
- CN-A- 103 536 288
- CN-A- 110 506 218
- CN-A- 113 805 114
- US-A1- 2012 191 383
- US-A1- 2012 191 383
- US-A1- 2019 155 709
- US-A1- 2019 347 177
- JAIN BHAVYA ET AL: "Image-Based Detection of MRI Hardware Failures : Algorithmen - Systeme - Anwendungen. Proceedings des Workshops vom 17. bis 19. März 2019 in Lübeck", BILDVERARBEITUNG FÜR DIE MEDIZIN 2019 : ALGORITHMEN - SYSTEME - ANWENDUNGEN. PROCEEDINGS DES WORKSHOPS VOM 17. BIS 19. März 2019 IN Lübeck, 1 January 2019 (2019-01-01), pages 206 - 211, XP093198110, DOI: 10.1007/978-3-658-25326-4_46
- KUHNERT NADINE ET AL: "Prediction of MRI Hardware Failures based on Image Features Using Time Series Classification", 1 January 2020 (2020-01-01), pages 131 - 136, XP093198116, Retrieved from the Internet <URL:https://arxiv.org/pdf/2001.02127> DOI: 10.1007/978-3-658-29267-6_27
- NADINE KUHNERT ET AL: "Prediction of MRI Hardware Failures based on Image Features using Ensemble Learning", ARXIV.ORG, CORNELL UNIVERSITY LIBRARY, 201 OLIN LIBRARY CORNELL UNIVERSITY ITHACA, NY 14853, 5 January 2020 (2020-01-05), XP081928680
- RÜCKER NADINE ET AL: "Hardware Failure Prediction on Imbalanced Times Series Data", JOURNAL OF DIGITAL IMAGING, vol. 34, no. 1, 6 January 2021 (2021-01-06), pages 182 - 189, XP037370355, ISSN: 0897-1889, DOI: 10.1007/S10278-020-00411-4

## Description

### TECHNICAL FIELD

The present disclosure generally relates to magnetic resonance imaging (MRI), and more particularly, relates to methods and systems for coil fault detection in MRI.

### BACKGROUND

An MRI device utilizes magnetic resonance (MR) coils to collect MR signals of a subject, and an MR image of the subject is generated based on the MR signals. The quality of the MR image may be negatively affected by a fault of the MR coils. Thus, it is desirable to provide methods and systems for efficiently determining whether an MR coil has a failure.

US 2019/155709 A1 discloses detecting a failure of a coil element of a multi-channel coil of an MRI system using a trained machine learning model.

### SUMMARY

An aspect of the present invention relates to a system for coil fault detection according to claim 1. The system includes at least one storage device including a set of instructions. The system also includes at least one processor in communication with the at least one storage device. When executing the set of instructions, the at least one processor is configured to cause the system to perform operations. The operations include obtaining one or more sets of reference signals collected by a coil of a magnetic resonance imaging (MRI) device in a pre-scan, the pre-scan being performed on a subject before an MRI scan of the subject. The coil includes multiple channels, wherein each of the multiple channels is configured to collect a portion of the one or more sets of reference signals. The operations also include obtaining a first fault detection model. The first fault detection model is a trained machine learning model. The operations include determining whether the coil has a failure by inputting the one or more sets of reference signals into the first fault detection model, wherein an output of the first fault detection model includes a determination result of whether the coil has a failure, and whether the coil has a failure is determined based on the determination result. In response to determining that the coil has a failure, a position of the failure of the coil is determined based on the output of the first fault detection model, wherein the position of the failure includes a serial number of a channel that has the failure among the plurality of channels, and the output further includes the serial number when the coil has a failure.

In some embodiments, the one or more sets of reference signals may include a set of MR signals collected in an acquisition that is performed on the subject after an excitation pulse is applied to the subject.

In some embodiments, the one or more sets of reference signals may include a set of noise signals collected in an acquisition that is performed on the subject without applying an excitation pulse to the subject.

In some examples that do not form part of the claimed invention, the determining whether the coil has a failure based on the one or more sets of reference signals and the first fault detection model may include determining a distribution of the set of noise signals, and determining whether the coil has a failure based on the distribution of the set of noise signals and the first fault detection model.

In some examples that do not form part of the claimed invention, the determining a distribution of the set of noise signals may include obtaining a set of reference noise signals, and determining, based on the set of noise signals and the set of reference noise signals, a probability distribution line representing the distribution of the set of noise signals in a two-dimensional probability space.

In some examples that do not form part of the claimed invention, the determining whether the coil has a failure based on the distribution of the set of noise signals and the first fault detection model may include determine a feature vector representing the probability distribution line, and determining whether the coil has a failure by inputting the feature vector into the first fault detection model.

In some examples that do not form part of the claimed invention, the first fault detection model may include a support vector machine (SVM) model. The determining whether the coil has a failure based on the distribution of the set of noise signals and the first fault detection model may include determining one or more decision boundaries based on the SVM model, determining a position of the probability distribution line relative to each of the one or more decision boundaries, and determining, whether the coil has a failure based on the position of the probability distribution line relative to each of the one or more decision boundaries.

In some examples that do not form part of the claimed invention, the obtaining a set of reference noise signals may include obtaining a plurality sets of reference noise signals. Each set of reference noise signals may be collected by a normal coil in an acquisition that is performed on a reference subject without applying an excitation pulse to the reference subject. The obtaining a set of reference noise signals may also include selecting the set of reference noise signals from the plurality sets of reference noise signals.

In some examples that do not form part of the claimed invention, the obtaining a set of reference noise signals may include obtaining a plurality of sets of preliminary noise signals. Each set of preliminary noise signals may be collected by a coil in an acquisition that is performed on a reference subject without applying an excitation pulse to the reference subject. The obtaining a set of reference noise signals may also include determining fitting parameters of a Weibull distribution model based on the plurality of sets of preliminary noise signals, and determining the set of reference noise signals based on the fitting parameters.

According to the invention, the system further includes in response to determining the coil has a failure, determining a position of the failure using the first fault detection model.

According to the invention, the coil includes a plurality of channels, and the position of the failure includes a serial number of a channel that has the failure among the plurality of channels.

In some embodiments, the system further may include in response to determining the coil has a failure, determining a type or a level of the failure using one or more second fault detection models different from the first fault detection model.

In some embodiments, the type of the failure may include at least one of a circuit disconnection, a failure of a receiving circuit, or a frequency offset of the coil.

In some embodiments, the system further may include obtaining device information of the MRI device, and determining whether the coil has a failure based on the one or more sets of reference signals, the first fault detection model, and the device information.

Another aspect of the present invention relates to a corresponding method according to claim 7.

Another aspect of the present invention relates to a corresponding non-transitory computer readable medium according to claim 10.

Additional features will be set forth in part in the description which follows, and in part will become apparent to those skilled in the art upon examination of the following and the accompanying drawings or may be learned by production or operation of the examples. The features of the present disclosure may be realized and attained by practice or use of various aspects of the methodologies, instrumentalities, and combinations set forth in the detailed examples discussed below. The scope of the invention is defined by the appended set of claims.

### BRIEF DESCRIPTION OF THE DRAWINGS

The present disclosure is further described in terms of exemplary embodiments. These exemplary embodiments are described in detail with reference to the drawings. The drawings are not to scale. These embodiments are non-limiting exemplary embodiments, in which like reference numerals represent similar structures throughout the several views of the drawings, and wherein:
FIG. 1 is a schematic diagram illustrating an exemplary fault detection system according to some embodiments of the present disclosure;
FIG. 2 is a schematic diagram illustrating hardware and/or software components of an exemplary computing device according to some embodiments of the present disclosure;
FIG. 3 is a block diagram illustrating an exemplary processing device according to some embodiments of the present disclosure;
FIG. 4A is a flowchart illustrating an exemplary process for coil fault detection according to some embodiments of the present disclosure;
FIG. 4B is a flowchart illustrating an exemplary process for determining whether a coil has a failure based on a set of noise signals according to some embodiments of the present disclosure;
FIG. 5 is a schematic diagram illustrating an exemplary distribution of a set of noise signals in a two-dimensional probability space according to some embodiments of the present disclosure;
FIG. 6 is a schematic diagram illustrating an exemplary distribution of a set of noise signals relative to one or more decision boundaries in a two-dimensional probability space according to some embodiments of the present disclosure;
FIG. 7 is an exemplary flowchart illustrating a training process of a first fault detection model according to some embodiments of the present disclosure;
FIG. 8 is an exemplary flowchart illustrating a training process of a second fault detection model according to some embodiments of the present disclosure;
FIG. 9 is a schematic diagram illustrating an exemplary interface of a terminal according to some embodiments of the present disclosure;
FIG. 10 is a schematic diagram illustrating another exemplary interface of a terminal according to some embodiments of the present disclosure; and
FIG. 11 is a schematic diagram illustrating an exemplary log according to some embodiments of the present disclosure.

### DETAILED DESCRIPTION

The following description is presented to enable any person skilled in the art to make and use the present disclosure and is provided in the context of a particular application and its requirements. Various modifications to the disclosed embodiments will be readily apparent to those skilled in the art, and the general principles defined herein may be applied to other embodiments and applications without departing from the scope of the present disclosure. The present invention is not limited to the embodiments shown but the scope of the invention is defined by the claims.

The terminology used herein is for the purpose of describing particular example embodiments only and is not intended to be limiting. As used herein, the singular forms "a," "an," and "the" may be intended to include the plural forms as well, unless the context clearly indicates otherwise. It will be further understood that the terms "comprise," "comprises," and/or "comprising," "include," "includes," and/or "including" when used in this disclosure, specify the presence of stated features, integers, steps, operations, elements, and/or components, but do not preclude the presence or addition of one or more other features, integers, steps, operations, elements, components, and/or groups thereof.

Provided herein are systems and components for medical imaging and/or medical treatment. In some embodiments, the medical system may include an imaging system. The imaging system may include a single modality imaging system and/or a multi-modality imaging system. The single modality imaging system may include, for example, a magnetic resonance imaging (MRI) system. Exemplary MRI systems may include a superconducting magnetic resonance imaging system, a non-superconducting magnetic resonance imaging system, etc. The multi-modality imaging system may include, for example, a magnetic resonance-computed tomography imaging (MRI-CT) system, a positron emission tomography-magnetic resonance imaging (PET-MRI) system, a single photon emission computed tomography-magnetic resonance imaging (SPECT-MRI) system, a digital subtraction angiography-magnetic resonance imaging (DSA-MRI) system, etc. In some embodiments, the medical system may include a treatment system. The treatment system may include a treatment plan system (TPS), image-guide radiotherapy (IGRT), etc. The image-guide radiotherapy (IGRT) may include a treatment device and an imaging device. The treatment device may include a linear accelerator, a cyclotron, a synchrotron, etc., configured to perform a radio therapy on a subject. The treatment device may include an accelerator of species of particles including, for example, photons, electrons, protons, or heavy ions.

According to the invention, one or more sets of reference signals are obtained. The set(s) of reference signals are collected by a coil of an MRI device in a pre-scan. The pre-scan is performed on a subject before an MRI scan of the subject. A first fault detection model in form of a trained machine learning model is also obtained. Whether the coil has a failure is determined based on the set(s) of reference signals and the first fault detection model. Therefore, the fault detection of the coil may be determined without acquiring an image of the coil (or each channel thereof), reducing the time for the fault detection of the coil and improving the efficiency of the fault detection of the coil. In addition, the fault detection may be implemented in real-time, and the failure of the coil may be detected timely, so that a maintenance personnel may maintain the coil in time, improving practicability and/or reliability. Moreover, the fault detection may be performed automatically without or with little user intervention, thereby reducing the workload of a user and avoiding cross-user variations.

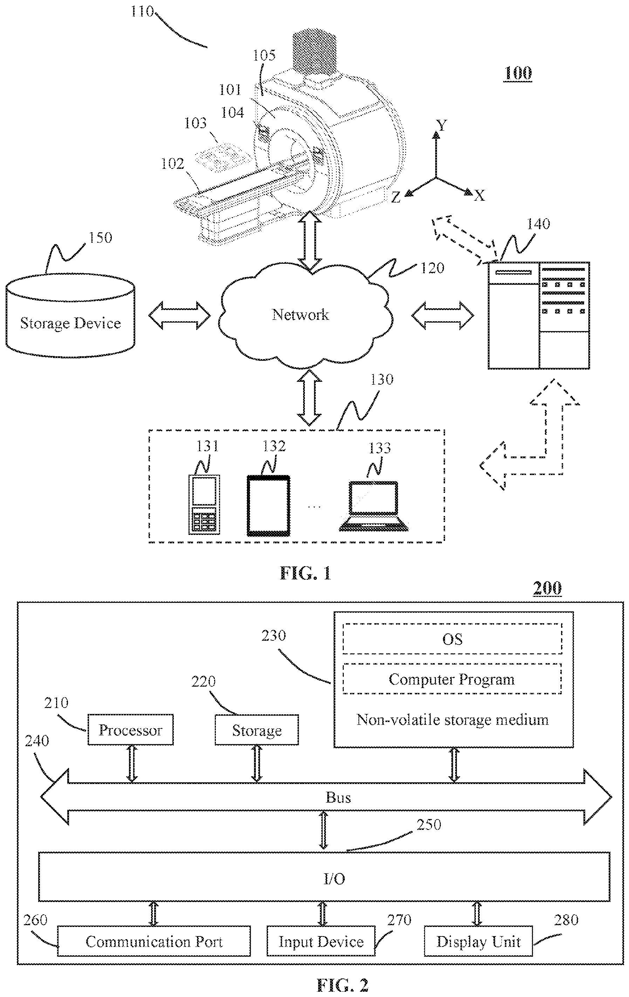

FIG. 1 is a schematic diagram illustrating an exemplary fault detection system 100 according to some embodiments of the present disclosure. The fault detection system 100 may be used to determine whether an MR coil has a failure. As illustrated, the fault detection system 100 may include an MRI device 110, a network 120, a terminal 130, a processing device 140, and a storage device 150. The components of the fault detection system 100 may be connected in one or more of various ways, for example, as indicated by arrows in FIG. 1.

The MRI device 110 may scan a subject located within its detection region and generate data relating to the subject. In the present disclosure, "subject" and "object" are used interchangeably. Merely by way of example, the subject may include a patient, a man-made subject, etc. As another example, the subject may include a specific portion, organ, and/or tissue of a patient. For example, the subject may include head, brain, neck, body, shoulder, arm, thorax, cardiac, stomach, blood vessel, soft tissue, knee, feet, or the like, or any combination thereof.

In the present disclosure, the X-axis, the Y-axis, and the Z-axis shown in FIG. 1 may form an orthogonal coordinate system. The X-axis and the Z-axis shown in FIG. 1 may be horizontal, and the Y-axis may be vertical. As illustrated, the positive X-direction along the X-axis may be from the left side to the right side of the MRI device 110 seen from the direction facing the front of the MRI device 110; the positive Y-direction along the Y-axis shown in FIG. 1 may be from the lower part to the upper part of the MRI device 110; the positive Z-direction along the Z-axis shown in FIG. 1 may refer to a direction in which the subject is moved out of the detection region (or referred to as the bore) of the MRI device 110.

In some embodiments, the MRI device 110 may include an MRI scanner 101, a patient support 102 (e.g., along the Z-direction), and a coil 103. The MRI scanner 101 may be configured to scan the subject located within its detection region and generate data relating to the subject. In some embodiments, the patient support 102 may be configured to support the subject. In some embodiments, the patient support 102 may have 6 degrees of freedom, for example, three translational degrees of freedom along three coordinate directions (i.e., X-direction, Y-direction, and Z-direction) and three rotational degrees of freedom around the three coordinate directions. Accordingly, the subject may be positioned by the patient support 102 within the detection region. Merely by way of example, the patient support 102 may move the subject into the detection region along the Z-direction in FIG. 1.

In some embodiments, the coil 103 may surround the subject. In some embodiments, the coil 103 may be movable and disposed on a surface of the patient support 102 or the subject. For example, the coil 103 may be disposed on the chest of the subject and attached to the subject through a strap or velcro to perform a cardiac scan of the subject. In some embodiments, the coil 103 may be configured to apply a radio frequency (RF) pulse to the subject. The RF pulse may excite nuclear spins inside the subject. In some embodiments, the coil 103 may be configured to receive a signal (also referred to as an MRI signal) of the nuclear spins inside the subject.

In some embodiments, the coil 103 may be served as a transmitter configured to apply the RF pulse, a receiver configured to receive the MRI signal, or both. In some embodiments, the coil 103 may include a body coil, a local coil, etc. In some embodiments, the coil may include a birdcage coil, a solenoid coil, a saddle coil, a Helmholtz coil, an array coil, a loop coil, etc. In some embodiments, the coil 103 may include a head coil, a spinal coil, an abdominal coil, an ankle coil, a wrist coil, a head-neck coil, a cervical-thoracic coil, a lower limb coil, a knee coil, etc.

In some embodiments, a gantry 105 may be located outside the MRI scanner 101 and configured to support a magnet (e.g., a main magnet), coils (e.g., gradient coils and/or radio frequency (RF) coils), etc. The gantry 105 may surround, along the Z-direction, the subject that is moved into or located within the detection region. In some embodiments, the MRI scanner 101 may include a bore. The patient support 102 may be configured to support the subject and move the subject inside or outside of the bore of the MRI scanner 101 for scanning. An accommodation space formed by the bore may be the detection region. A middle portion of the detection region may be located at a central region of the main magnet. An imaging effect of the middle portion of the detection region may be better than an imaging effect of two sides of the detection region. In some embodiments, the MRI scanner 101 may be a close-bore scanner or an open-bore scanner. In some embodiments, a posture of the subject disposed on the patient support 102 may include a supine posture, a left lateral posture, a right lateral posture, a prone posture, etc.

In some embodiments, the main magnet (e.g., a resistive magnet, a superconductive magnet, a permanent magnet) may generate a first magnetic field (or referred to as a main magnetic field) that may be applied to a subject positioned inside the first magnetic field. The gradient coils may generate a second magnetic field (or referred to as a gradient field, including gradient fields Gx, Gy, and Gz). The second magnetic field may be superimposed on the main magnetic field generated by the main magnet and distort the main magnetic field so that the magnetic orientations of the protons of a subject may vary as a function of their positions inside the gradient field, thereby encoding spatial information into MR signals generated by the region of the subject being imaged. The gradient coils may include X coils (e.g., configured to generate the gradient field Gx corresponding to the X-direction), Y coils (e.g., configured to generate the gradient field Gy corresponding to the Y-direction), and/or Z coils (e.g., configured to generate the gradient field Gz corresponding to the Z-direction). The three sets of coils may generate three different magnetic fields that are used for position encoding. The gradient coils may allow spatial encoding of MR signals for image reconstruction.

In some embodiments, a display 104 may be mounted at the gantry 105 (e.g., a surface thereof). The display 104 may be connected to the processing device 140 and/or the terminal 130. The display 104 may be configured to display a working status of the MRI device 110, information of the subject, a protocol provided to a physician, etc. It should be understood that, in some embodiments, the display 104 may be connected to the MRI scanner 101 through a wire connection or a wireless connection to receive a signal collected by the coil 103. For example, the display 104 may be connected to a controller of the MRI device 110 to receive a cardiac motion curve or a signal determined by the controller. In some embodiments, the working status of the MRI device 110 may include a scanning sequence, a working status of a magnet, a working status of a gradient, a scanning time, a human-specific absorption rate, fault detection information of the coil 103, etc. For example, the information of the subject may include a height, a weight, a gender, a portion to be scanned, a respiratory motion status, a cardiac motion status, etc. The protocol provided to the physician may be displayed in a form of a list, a humanoid figure, or a combination thereof.

The "patient support" disclosed in the present disclosure may refer to any structure supporting the subject, and can be used interchangeably with other terms such as "couch," "scanning bed," "supporting table," "supporting bed," "detection bed," etc. The "scanner" disclosed in the present disclosure may be used interchangeably with other terms such as "scanning device," "scanning system," "scanning apparatus," "image scanning device," "imaging device," etc. The subject may include a human body, an animal body, a water phantom, or other living and non-living subjects.

The network 120 may include any suitable network that can facilitate the exchange of information and/or data for the fault detection system 100. In some embodiments, one or more components of the fault detection system 100 (e.g., the MRI device 110, the terminal 130, the processing device 140, or the storage device 150) may communicate information and/or data with one or more other components of the fault detection system 100 via the network 120. For example, the processing device 140 may obtain one or more sets of reference signals from the MRI device 110 via the network 120. In some embodiments, the network 120 may be any type of wired or wireless network, or a combination thereof.

The terminal 130 may include a mobile device 131, a tablet computer 132, a laptop computer 133, or the like, or any combination thereof. In some embodiments, the terminal 130 may remotely operate the MRI device 110 and/or the processing device 140. In some embodiments, the terminal 130 may be part of the processing device 140. In some embodiments, the terminal 130 may be omitted.

The processing device 140 may process data and/or information obtained from the MRI device 110, the terminal 130, and/or the storage device 150. For example, the processing device 140 may determine whether a coil of the MRI device 110 has a failure based on a first fault detection model. In some embodiments, the processing device 140 may be a single server or a server group. The server group may be centralized or distributed. In some embodiments, the processing device 140 may be local or remote. In some embodiments, the processing device 140 may be implemented on a cloud platform.

The storage device 150 may store data and/or instructions. In some embodiments, the storage device 150 may store data obtained from the MRI device 110, the terminal 130 and/or the processing device 140. For example, the storage device 150 may store one or more sets of reference signals collected by a coil of the MRI device 110 in a pre-scan and/or a fault detection model (e.g., a first fault detection model, a second fault detection model). In some embodiments, the storage device 150 may store data and/or instructions that the processing device 140 may execute or use to perform exemplary methods described in the present disclosure. For example, the storage device 150 may store instructions that the processing device 140 may execute to determine whether a coil of the MRI device 110 has a failure. In some embodiments, the storage device 150 may include a mass storage device, a removable storage device, a volatile read-and-write memory, a read-only memory (ROM), or the like, or any combination thereof. In some embodiments, the storage device 150 may be implemented on a cloud platform.

FIG. 2 is a schematic diagram illustrating hardware and/or software components of an exemplary computing device according to some embodiments of the present disclosure. The computing device 200 may be used to implement any component of the fault detection system 100 as described herein. For example, the processing device 140 and/or the terminal 130 may be implemented on the computing device 200, respectively, via its hardware, software program, firmware, or a combination thereof. As illustrated in FIG. 2, the computing device 200 may include a processor 210, a storage 220, a non-volatile storage medium 230, a bus 240, an input/output (I/O) 250, a communication port 260, an input device 270, and a display unit 280.

The processor 210 may execute computer instructions (program code) and perform functions of the processing device140 in accordance with techniques described herein. For example, the processor 210 may determine whether a coil of the MRI device 110 has a failure.

The storage 220 may store data/information obtained from the MRI device 110, the terminal 130, the storage device 150, or any other component of the fault detection system 100. For example, the storage 220 may store a program for the processing device 140 for determining whether a coil of the MRI device 110 has a failure.

The non-volatile storage medium 230 may store an operating system (OS) and a computer program. In some embodiments, the operating system (e.g., iOS, Windows, etc.) and the computer program may be loaded into the non-volatile storage medium 230 from the storage 220 in order to be executed by the processor 210.

The bus 240 may transmit signals, data, or information. The processor 210, the storage 220, the non-volatile storage medium 230, the I/O 250, the communication port 260, the input device 270, and/or the display unit 280 may be connected to the bus 240 to be in communication with each other.

The I/O 250 may input or output signals, data, or information. In some embodiments, the I/O 250 may enable user interaction with the processing device 140. Exemplary input devices 270 may include a keyboard, a mouse, a touch screen, a microphone, a trackball, or the like, or a combination thereof. Exemplary display unit 280 may include a liquid crystal display (LCD), a light-emitting diode (LED)-based display, a flat panel display, a curved screen, a television device, a cathode ray tube (CRT), or the like, or a combination thereof.

The communication port 260 may be connected to a network (e.g., the network 120) to facilitate data communications. The communication port 260 may establish connections between the processing device140, the MRI device 110, the terminal 130, or the storage device 150.

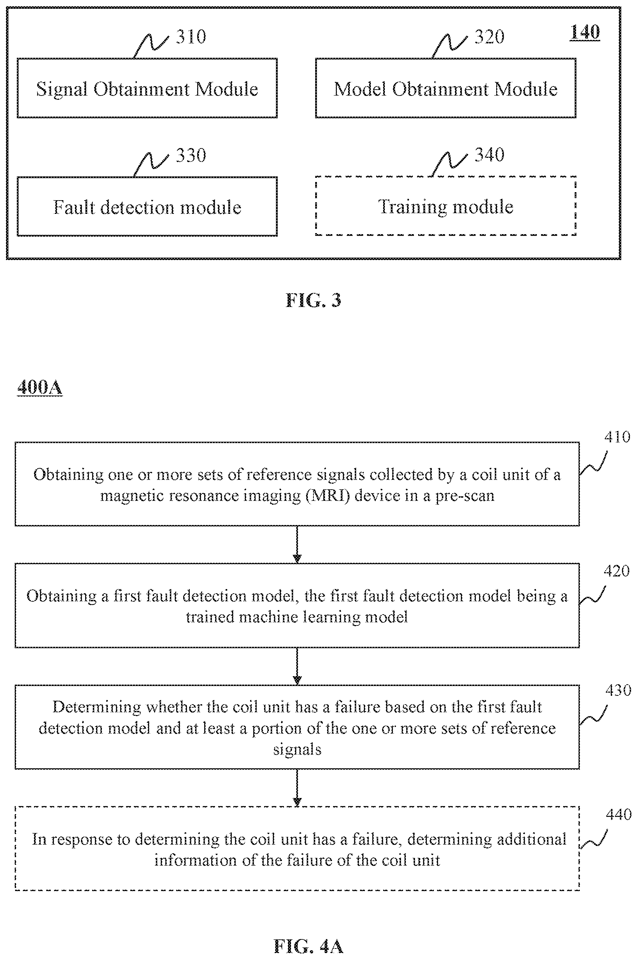

FIG. 3 is a schematic block diagram illustrating an exemplary processing device according to some embodiments of the present disclosure. The processing device 140 may include a signal obtainment module 310, a model obtainment module 320, and a fault detection module 330.

The signal obtainment module 310 may be configured to obtain one or more sets of reference signals collected by a coil of a magnetic resonance imaging (MRI) device (e.g., the MRI device 110) in a pre-scan. More descriptions of the obtainment of the one or more sets of reference signals may be found elsewhere in the present disclosure (e.g., operation 410 or the descriptions thereof).

The model obtainment module 320 may be configured to obtain a first fault detection model (e.g., a trained machine learning model). More descriptions of the obtainment of the first fault detection model may be found elsewhere in the present disclosure (e.g., operation 420 or the descriptions thereof).

The fault detection module 330 may be configured to determine whether the coil has a failure based on the first fault detection model and at least a portion of the one or more sets of reference signals. More descriptions of determining whether the coil has a failure may be found elsewhere in the present disclosure (e.g., operation 430 or descriptions thereof).

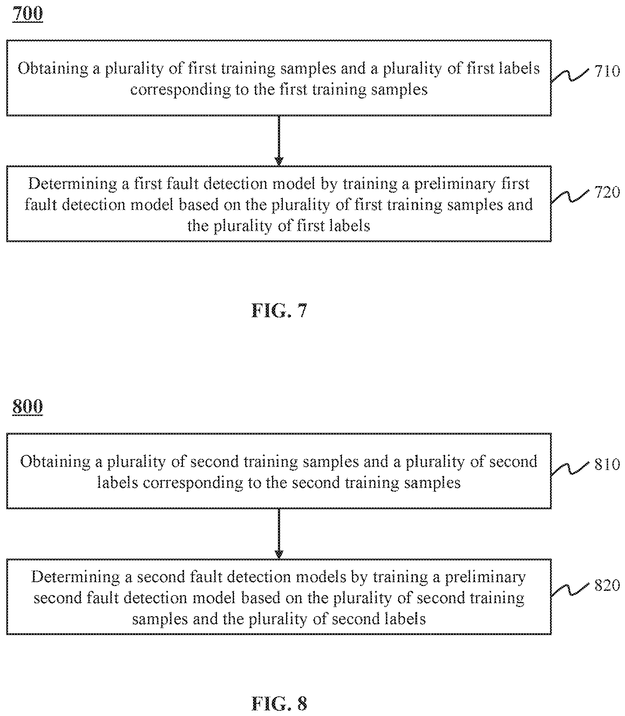

In some embodiments, the processing device 140 may also include a training module 340. The training module 340 may be configured to determine a model (e.g., the first fault detection model, one or more second fault detection models) by training a preliminary model based on a plurality of training samples, more descriptions of which may be found elsewhere in the present disclosure (e.g., FIG. 7, FIG. 8, or the descriptions thereof). In some embodiments, the first fault detection model may be generated by a device external to the processing device 140 (e.g., a processing device of a vendor of the first fault detection model).

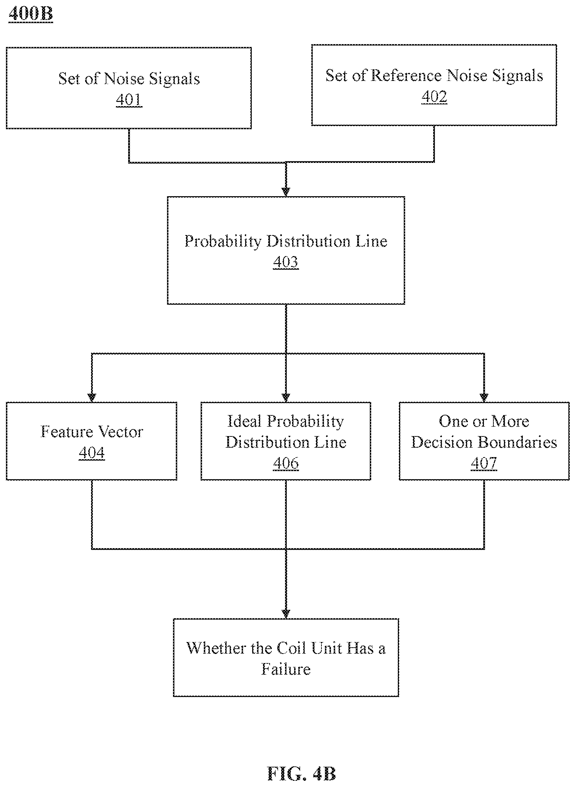

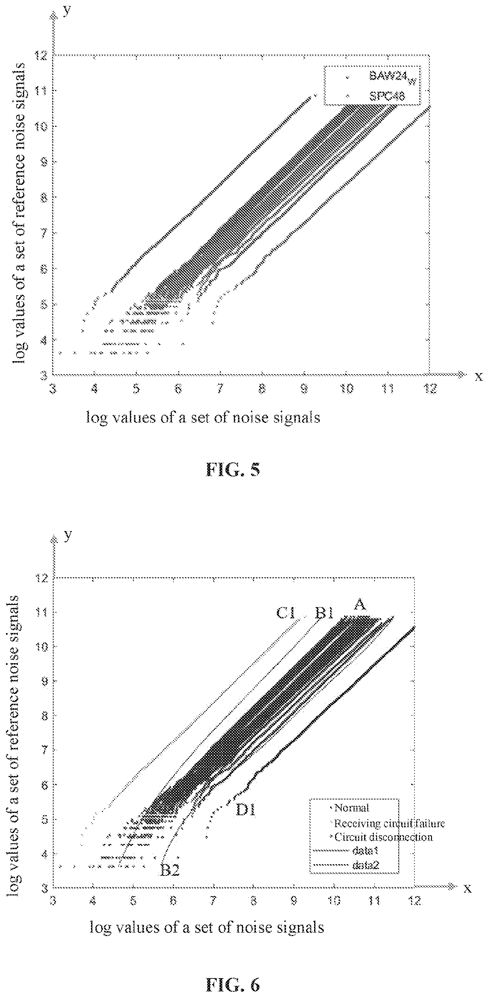

FIG. 4A is a flowchart illustrating an exemplary process for coil fault detection according to some embodiments of the present disclosure. FIG. 4B is a flowchart illustrating an exemplary process for determining whether a coil has a failure based on a set of noise signals according to some embodiments of the present disclosure. FIG. 5 is a schematic diagram illustrating an exemplary distribution of a set of noise signals in a two-dimensional probability space according to some embodiments of the present disclosure. FIG. 6 is a schematic diagram illustrating an exemplary distribution of a set of noise signals relative to one or more decision boundaries in a two-dimensional probability space according to some embodiments of the present disclosure.

In 410, the processing device 140 (e.g., the signal obtainment module 310) may obtain one or more sets of reference signals (also referred to as pre-scan signals) collected by a coil (e.g., the coil 103) of an MRI device (e.g., the MRI device 110) in a pre-scan.

In some embodiments, the pre-scan may be performed on a subject before an MRI scan of the subject. In some embodiments, the pre-scan may be used to collect information for MRI device calibration, noise information (e.g., a noise coupling matrix), etc. For example, the MRI device calibration may include a calibration of coil sensitivity, a calibration of magnetic field homogeneity, a frequency calibration of a main magnetic field, a calibration of a voltage of the main magnetic field, a calibration of brightness homogeneity, a deformation calibration, or the like, or any combination thereof. In some embodiments, the MRI scan may be performed after the MRI device calibration. The MRI scan may be used to generate an MR image of the subject. The MR image may be used for disease diagnosis of the subject.

In some embodiments, the pre-scan may be unnecessary to be performed before each MRI scan. Taking a first MRI scan and a second MRI scan after the first MRI scan as an example, if parameters of the MRI device for the second MRI scan remain the same as the first MRI scan and if the pre-scan has been performed before the first MRI scan, the pre-scan before the second MRI scan may be unnecessary. Exemplary parameters of the MRI device may include a field of view (FOV), coil sensitivity, magnetic field homogeneity, a frequency of the main magnetic field, or the like, or any combination thereof. For example, the pre-scan may be performed before MRI scans of different FOVs, respectively. As another example, for different MRI scans of the same FOV, the pre-scan may be necessary to be performed before only one MRI scan of the different MRI scans.

In some embodiments, in order to obtain the one or more sets of reference signals, multiple pre-scans may need to be performed. In some embodiments, the one or more sets of reference signals may include a set of MR signals collected in an acquisition that is performed on the subject after an excitation pulse (e.g., a radio frequency pulse) is applied to the subject. The set of MR signals may include a free induction decay (FID) signal, a spin echo (SE) signal, a stimulated echo (STE) signal, or the like, or any combination thereof.

In some embodiments, after the excitation pulse (e.g., a 90° pulse) is appliedto the subject, the macroscopic transverse magnetization may be generated in tissues of the subject. After the excitation pulse terminates, due to factors such as T2 relaxation and the inhomogeneity of the main magnetic field, the macroscopic transverse magnetization in the tissues may decay exponentially, that is, have a free induction decay. In some embodiments, the FID signal may be obtained by recording the free induction decay of the transverse magnetization directly using the coil.

In some embodiments, after the excitation pulse (e.g., a 90° pulse) is appliedto the subject, the macroscopic transverse magnetization may be generated in tissues of the subject. After the excitation pulse terminates, the inhomogeneity of the main magnetic field may cause protons to dephase, and the macroscopic transverse magnetization in the tissues may decay gradually. If a refocusing pulse (e.g., a 180°pulse) is applied to the subject, the protons may be gradually rephased, and the macroscopic transverse magnetization in the tissues may reach the maximum value. Then the protonsare gradually dephased, and the macroscopic transverse magnetization in the tissues may gradually decay. In some embodiments, the SE signal may be obtained by recording the above changing process of the transverse magnetization directly using the coil.

In some embodiments, the stimulated echo may be obtained by a stimulated echo acquisition mode (STEAM). The STEAM may include three slice-selective pulses (e.g., 90° excitation pulses), and each pulse may be successively applied to the subject by an orthogonal gradient. The SE signal may be generated within a voxel where the three slices intersect. The first slice-selective pulse may cooperate with a slice selection gradient to excite all nuclear protons in the selected slice. Under the action of the second slice-selective pulse, a magnetization vector in a first plane (e.g., the XY plane) is flipped and lies in a second plane (e.g., the XZ plane) perpendicular to the first plane. The third slice-selective pulse may flip all nuclear protons into the first plane and rephase to form the echo again through half echo time (TE). In some embodiments, the STE signal may be obtained by recording the stimulated echo directly using the coil.

In some embodiments, the one or more sets of reference signals may include a set of noise signals collected in an acquisition that is performed on the subject without applying an excitation pulse to the subject. The noise signals may include signals of background noise (e.g., environmental noise), which may interfere with normal MR signals collected by an MRI scan, thereby reducing the imaging quality. In some embodiments, the noise signals may represent a standard deviation of the intensity of noises collected by the coil.

According to the invention, the coil (e.g., an array coil) includes multiple channels (also referred to as coil units), for example, 4 channels, 8 channels, 16 channels, 24 channels, 32 channels, 64 channels, 128 channels, etc. Each of the multiple channels is configured to collect a portion of the one or more sets of reference signals (e.g., one set of reference signals). More descriptions of the coil may be found elsewhere in the present disclosure, for example, FIG. 1 or the descriptions thereof.

In some embodiments, the processing device 140 may obtain the one or more sets of reference signals from one or more components (e.g., the MRI device 110, the terminal 130, and/or the storage device 150) of the fault detection system 100 or an external storage device via the network 120. For example, the processing device 140 may instruct the MRI device 110 to acquire the one or more sets of reference signals and transmit the set(s) of reference signals to the storage device 150, or any other storage device for storage. The processing device 140 may obtain the one or more sets of reference signals from the storage device 150 or any other storage device. As another example, the processing device 140 may obtain the one or more sets of reference signals from the MRI device 110 directly.

In 420, the processing device 140 (e.g., the model obtainment module 320) may obtain a first fault detection model. The first fault detection model may be configured to determine whether the coil has a failure. In some embodiments, the failure of the coil may include a circuit disconnection, a failure of a receiving circuit, a frequency offset of the coil, or the like, or any combination thereof. The circuit disconnection may be caused by a breakage of a flexible board. The failure of a receiving circuit may include a disconnection of the receiving circuit caused by a malfunction of an amplifier connected with the receiving circuit, a damage of a connection pin of the coil, or a cable disconnection, etc. The frequency offset of the coil may be caused by a damaged capacitor contained in the coil, a diode failure, etc. In some embodiments, the processing device 140 may determine the frequency offset of the coil based on at least a portion of the set of MR signals.

According to the invention, in response to determining that the coil has a failure, the first fault detection model is configured to determine a position of the failure. According to the invention, the coil includes a plurality of channels, and the position of the failure includes a serial number of a channel that has the failure among the plurality of channels. More descriptions of the utilization of the first fault detection model may be found elsewhere in the present disclosure (e.g., operation 430 or the descriptions thereof).

In some embodiments, the first fault detection model may be a trained machine learning model (e.g., a support vector machine (SVM) model). More descriptions of the training of the first fault detection model may be found elsewhere in the present disclosure (e.g., FIG. 7, or the descriptions thereof).

In some embodiments, the processing device 140 may obtain one or more second fault detection models different from the first fault detection model. The one or more second fault detection models may be configured to determine a type of the failure, a level of the failure, etc. The type of the failure may include a circuit disconnection, a failure of a receiving circuit, a frequency offset of the coil, etc. In some embodiments, each of the one or more second fault detection models may be configured to detect a failure of a certain type. For example, the one or more second fault detection models may include a model for detecting a circuit disconnection, a model for detecting a failure of a receiving circuit, a model for detecting a frequency offset of the coil, or the like, or any combination thereof.

In some embodiments, the level of a failure may represent the severity of the failure. In some embodiments, the larger the level is, the severer the failure may be. More descriptions of the utilization of the one or more second fault detection models may be found elsewhere in the present disclosure (e.g., operation 430 or the descriptions thereof).

In some embodiments, a second fault detection model may be a trained machine learning model (e.g., a support vector machine (SVM) model). More descriptions of the training of a second fault detection model may be found elsewhere in the present disclosure (e.g., FIG. 8 or the descriptions thereof).

It should be noted that the above descriptions are for illustration purposes and are non-limiting. In some embodiments, the functions of the one or more second fault detection models may be achieved by a single model that is configured to determine failures of different types. In some embodiments, the functions of the first fault detection model and the one or more second fault detection models may be achieved by a single model that is configured to determine whether the coil has a failure, determine the position of the failure, and additional information (e.g., the type) of the failure.

In some embodiments, the processing device 140 may obtain the first fault detection model, the one or more second fault detection model, and/or the single model from one or more components (e.g., the terminal 130, and/or the storage device 150) of the fault detection system 100 or an external storage device via the network 120.

In 430, the processing device 140 (e.g., the fault detection module 330) may determine whether the coil has a failure based on the first fault detection model and at least a portion of the one or more sets of reference signals.

In some embodiments, the at least a portion of the one or more sets of reference signals may include at least a portion of the set of MR signals, at least a portion of the set of noise signals, or a combination thereof. For example, the processing device 140 may determine whether the coil has the failure based on at least a portion of the set of MR signals, e.g., the FID signal, the SE signal, the STE signal, or any combination thereof. As another example, the processing device 140 may determine whether the coil has a failure based on at least a portion of the set of noise signals. As a further example, the processing device 140 may determine whether the coil has a failure based on at least a portion of the set of MR signals and at least a portion of the set of noise signals. According to some embodiments of the present disclosure, the at least a portion of the one or more sets of reference signals may be selected for the fault detection of the coil according to practical demands, improving the practicability of the fault detection.

According to the invention, the processing device 140 determines whether the coil has a failure by inputting the at least a portion of the one or more sets of reference signals into the first fault detection model. The first fault detection model outputs a determination result of whether the coil has a failure or, in examples that do not form part of the claimed invention, a probability that the coil has a failure, or the like, or any combination thereof. In examples not forming part of the claimed invention, the processing device 140 may preprocess the at least a portion of the one or more sets of reference signals. The processing device 140 may determine whether the coil has a failure by inputting the preprocessed at least a portion of the one or more sets of reference signals (e.g., a distribution, a probability distribution line, a feature vector as described below) into the first fault detection model.

In some embodiments, the processing device 140 may determine a distribution of the set of noise signals, and then determine whether the coil has a failure based on the distribution of the set of noise signals and the first fault detection model. For example, the processing device 140 may determine whether the coil has a failure by inputting the distribution of the set of noise signals into the first fault detection model. In some embodiments, the processing device 140 may determine a probability distribution line representing the distribution of the set of noise signals in a two-dimensional probability space (e.g., as shown in FIG. 5 or FIG. 6). The processing device 140 may determine whether the coil has a failure based on the probability distribution line and the first fault detection model. For example, the processing device 140 may determine whether the coil has a failure by inputting the probability distribution line and/or information relating to the probability distribution line (e.g., a feature vector representing the probability distribution line) into the first fault detection model.

In some embodiments, device information of the MRI device may affect the fault detection of the coil. The processing device 140 may obtain device information of the MRI device, The processing device 140 may determine whether the coil has a failure based on the first fault detection model, the device information, and at least a portion of the one or more sets of reference signals, thereby improving the accuracy of the fault detection of the coil. The processing device 140 may determine whether the coil has a failure by inputting the device information together with information described above (e.g., the at least a portion of the one or more sets of reference signals, the distribution, the probability distribution line, the feature vector) into the first fault detection model. In some embodiments, the device information may include coil information, magnetic field information, or the like, or any combination thereof. The coil information may include a size of a coil, a count of turns of the coil, a material of the coil, a type of the coil, a count of channels of the coil, or the like, or any combination thereof. The magnetic field information may include an intensity of the main magnetic field. In some embodiments, the processing device 140 may obtain the device information from one or more components (e.g., the MRI device 110, the terminal 130, and/or the storage device 150) of the fault detection system 100 or an external storage device via the network 120.

It should be noted that the above descriptions are for illustration purposes and are non-limiting. In some embodiments, there may be different first fault detection models corresponding to different types of devices (e.g., different devices of different device information). Each of the different first fault detection models may be used to implement the failure detection of the device of a predetermined type.

In some embodiments, environmental information of the MRI device may affect the fault detection of the coil. The processing device 140 may obtain the environmental information of the MRI device. The processing device 140 may determine whether the coil has a failure based on the first fault detection model, the environmental information, and at least a portion of the one or more sets of reference signals, thereby improving the accuracy of the fault detection of the coil. The processing device 140 may determine whether the coil has a failure by inputting the environmental information together with information described above (e.g., the at least a portion of the one or more sets of reference signals, the distribution, the probability distribution line, the feature vector, the device information) into the first fault detection model. In some embodiments, the environmental information may include information relating to electromagnetic interference (EMI). EMI may interfere with signals collected by the coil of the MRI device, and an accuracy of the fault detection of the coil may be reduced due to the interference. For example, if a walkie-talkie is used near the MRI, the coil of the MRI device may receive a radio wave (e.g., 128MHz) relating to the walkie-talkie. The radio wave of 128 MHz may be superimposed with random noise signals with small amplitudes in the environment collected by the coil, which may increase a difficulty of detecting a failure of the coil. In some embodiments, the processing device 140 may obtain the environmental information from one or more components (e.g., the MRI device 110, the terminal 130, and/or the storage device 150) of the fault detection system 100 or an external storage device via the network 120.

In some embodiments, process 400B as illustrated in FIG. 4B may be performed to determine whether the coil has a failure based on the set of noise signals.

In 401, the processing device 140 may obtain the set of noise signals.

In 402, the processing device 140 may obtain a set of reference noise signals. The set of reference noise signals may represent ideal noise signals collected by a normal coil in an acquisition that is performed on a reference subject without applying an excitation pulse to the reference subject.

In some embodiments, the processing device 140 may obtain a plurality of sets of reference noise signals. Each set of reference noise signals may be collected by a normal coil in an acquisition that is performed on a reference subject without applying an excitation pulse to the reference subject. A noise signal database may be established based on the sets of reference noise signals. In some embodiments, the processing device 140 may select the set of reference noise signals from the plurality sets of reference noise signals. For example, the processing device 140 may designate one set of the plurality sets of reference noise signals, which are collected by an MRI device of same parameters (e.g., the intensity of the main magnetic field, a count of channels included in a coil) as the MRI device used to collect the set of noise signals, as the set of reference noise signals.

In some embodiments, the intensity of the main magnetic field may affect noise signals. The processing device 140 may obtain different groups of sets of reference noise signals. Each group of the plurality sets of reference noise signals may be collected at a predetermined intensity (e.g., 1.5 T, 3.0 T, 5.0 T) of the main magnetic field. For example, the groups of sets of reference noise signals may include a group of sets of reference noise signals collected at 1.5 T, a group of sets of reference noise signals collected at 3.0 T, a group of sets of reference noise signals collected at 5.0 T. In some embodiments, if the set of noise signals is collected at a specific intensity of the main magnetic field, the processing device 140 may select one set of reference noise signals from a group of sets of reference noise signals corresponding to the specific intensity of the main magnetic field as the set of reference noise signals.

In some embodiments, the processing device 140 may obtain a plurality of sets of preliminary noise signals. Each set of preliminary noise signals may be collected by a coil (being normal or abnormal) in an acquisition that is performed on a reference subject without applying an excitation pulse to the reference subject. The processing device 140 may determine fitting parameters of a Weibull distribution model based on the plurality of sets of preliminary noise signals. The processing device 140 may determine the set of reference noise signals based on the fitting parameters. In some embodiments, the processing device 140 may determine a histogram of each set of preliminary noise signals that is approximate with the Weibull distribution model. The processing device 140 may determine the fitting parameters of the Weibull distribution model based on a plurality of histograms corresponding to the plurality of sets of preliminary noise signals. In other words, ideal noise signals collected by a normal coil in an acquisition without an excitation pulse may be estimated based on the Weibull distribution model.

In 403, the processing device 140 may determine a probability distribution line representing the distribution of the set of noise signals in a two-dimensional probability space based on the set of noise signals and the set of reference noise signals. For example, the processing device 140 may determine the two-dimensional probability space based on the set of noise signals and the set of reference noise signals, and determine the probability distribution line corresponding to the set of noise signals in the two-dimensional probability space. In some embodiments, the two-dimensional probability space may include a horizontal axis (e.g., x axis in FIG. 5 or FIG. 6) and a vertical axis (e.g., y axis in FIG. 5 or FIG. 6). The horizontal axis may represent an intensity of a noise signal, and the vertical axis may represent an intensity of a reference noise signal. In some embodiments, the processing device 140 may sort the set of noise signals according to their respective signal intestines, and perform a log operation on the set of noise signals to determine the horizontal coordinates of the points in the two-dimensional probability space. The processing device 140 may also sort the set of reference noise signals according to their respective signal intestines, and perform the log operation on the set of reference noise signals to determine the vertical coordinates of the points in the two-dimensional probability space. The sorted noise signals and sorted reference noise signals may form a line (defined as a probability distribution line) by connecting the points in the two-dimensional probability space. The probability distribution line may be used to represent the probability distribution of the sorted noise signal and reference noise signal.

In some embodiments, the processing device 140 may determine the probability distribution line based on a Weibull distribution model. The processing device 140 may determine fitting parameters of the Weibull distribution model based on the set of noise and/or the set of reference noise signals, and determine the probability distribution line based on the fitting parameters.

In some embodiments, the coil may include a plurality of channels. For each of the plurality of channels, the processing device 140 may determine a distribution of a portion of the set of noise signals collected by the channel and a probability distribution line representing the distribution.

Whether the coil has a failure may be determined based on the probability distribution line via various manners.

In some embodiments, operation 404 may be performed. In 404, the processing device 140 may determine a feature vector representing the probability distribution line. For example, the feature vector may include a two-dimensional feature vector constructed based on a slope and an intercept of the probability distribution line or a multi-dimensional feature vector constructed based on the plurality of points on the probability distribution line. Then, the processing device 140 may determine whether the coil has a failure based on the feature vector. For example, the feature vector may be input into the first fault detection model, and the first fault detection model may output a determination result of whether the coil has a failure or a probability that the coil has a failure.

In some embodiments, operation 406 may be performed. In 406, the processing device 140 may determine an ideal probability distribution line representing an ideal distribution of noise signals collected by a normal coil. The ideal probability distribution line may (substantially) coincide with a line passing through an origin point (i.e., an origin point of the two-dimensional probability space) of the horizontal axis and the vertical axis, and an angle between the line and the horizontal axis or the vertical axis may be 45°, that is, the ideal probability distribution line is y=x. As used herein, substantially, when used to qualify a feature (e.g., equivalent to, the same as, etc.), indicates that the deviation from the feature is below a threshold, e.g., 30%, 25%, 20%, 15%, 10%, 5%, etc.

Further, the processing device 140 may determine whether the coil has a failure based on the probability distribution line and the ideal probability distribution line. In some embodiments, the processing device 140 may determine a distance between the probability distribution line and the ideal probability distribution line, and determine whether the coil has a failure based on the distance. A distance between the probability distribution line and the ideal probability distribution line may be, for example, a distance from a point on the probability distribution line to a corresponding point on the ideal probability distribution line, an average distance (or a median distance or a maximum distance) from all points on the probability distribution line to their respective corresponding points on the ideal probability distribution line. In some embodiments, the point may correspond to an intensity exceeding an intensity threshold. In some embodiments, the processing device 140 may determine whether the coil has a failure by comparing the distance between the probability distribution line and the ideal probability distribution line with a distance threshold. In response to determining that the distance is less than or equal to the distance threshold, the coil may be deemed as being normal (i.e., the coil doesn't have a failure). In response to determining that the distance is greater than the distance threshold, the processing device 140 may determine that the coil has a failure.

In some embodiments, the larger the distance between the probability distribution line and the ideal probability line, the severer the failure of the coil may be. Accordingly, the processing device 140 may also determine a level of the failure (mild, medium severe) based on the distance. There may be different distance thresholds used to determine different failure levels. For example, in response to determining the distance is smaller than or equal to a first distance threshold, the processing device 140 may determine that the coil has a mild failure; in response to determining the distance is larger than the first distance threshold and smaller than or equal to a second distance threshold, the processing device 140 may determine that the coil has a medium failure; in response to determining the distance is larger than the second distance threshold, the processing device 140 may determine that the coil has a severe failure. The first distance threshold may be smaller than the second distance threshold.

In some embodiments, the first fault detection model may include a support vector machine (SVM) model, and operation 407 may be performed. In 407, the processing device 140 may determine one or more decision boundaries based on the SVM model. Each of the one or more decision boundaries may be used to determine whether the coil has a failure of a predetermined type. Further, the processing device 140 may determine a position of the probability distribution line relative to each of the one or more decision boundaries in the two-dimensional probability space, and determine whether the coil has a failure based on the position of the probability distribution line relative to each decision boundary. In response to determining the coil has a failure, the processing device 140 may also determine a type of the failure based on the position of the probability distribution line relative to each decision boundary. Merely by way of example, for a first decision boundary located at the left of the ideal probability distribution line, in response to determining the probability distribution line is at the left side of the first decision boundary in the two-dimensional probability space, the processing device 140 may determine that the coil has a failure and/or the failure is of a first type. As another example, for a second decision boundary located at the right of the ideal probability distribution line, in response to determining the probability distribution line is at the right side of the second decision boundary in the two-dimensional probability space, the processing device 140 may determine that the coil has a failure and/or the failure is of a second type. As yet another example, in response to determining the probability distribution line between the first decision boundary and the second decision boundary, the processing device 140 may determine that the coil is normal.

For illustration purposes, FIG. 6 illustrates a first decision boundary B1 corresponding to a first type of failure and a second decision boundary B2 corresponding to a second type of failure. A portion of the first decision boundary B1 (intensities thereof exceeding the intensity threshold) may be located at the left of the ideal probability distribution line, and the second decision boundary B2 may be located at the right side of the ideal probability distribution line. A first probability distribution line C1 is at the left side of the first decision boundary B1 in the probability space, so the processing device 140 may determine that the coil has a failure and/or the failure is of the first type. A second probability distribution line D1 is at the right side of the second decision boundary B2 in the probability space, so the processing device 140 may determine that the coil has a failure and the failure is of the second type.

In some embodiments, the first decision boundary may be used to detect a circuit disconnection. The second decision boundary may be used to detect a failure of a receiving circuit. Thus, the coil (or a channel thereof) corresponding to the first probability distribution line C1 may have a failure of circuit disconnection. The coil (or a channel thereof) corresponding to the second probability distribution line D1 may have a failure of the receiving circuit.

It should be noted that the above descriptions are for illustration purposes and are non-limiting. In some embodiments, the processing device 140 may determine fitting parameters of the Weibull distribution model based on the set of noise signals, and determine whether the coil has a failure by inputting the fitting parameters into the first fault detection model. For example, the fitting parameters may include two parameters, the processing device 140 may construct a two-dimensional feature vector based on the fitting parameter, and determine whether the coil has a failure by inputting the feature vector into the first fault detection model.

In some embodiments, as shown in FIG. 4A, in response to determining the coil has a failure, operation 440 may be performed. In 440, the processing device 140 may determine additional information of the failure of the coil. The additional information of the failure of the coil may include a type of the failure, a level of the failure, or the like, or a combination thereof. According to the invention, the coil includes a plurality of channels, and each of the plurality of channels is configured to collect a portion of the one or more sets of reference signals. The position of the failure includes a position of the channel. In some embodiments, each of the plurality of channels may have a serial number indicating the position of the channel. The position of the failure includes a serial number of a channel that has a failure. In some embodiments, the level of the failure may indicate the severity of the failure. In some embodiments, the larger the level of the failure is, the severer the failure may be.

In some embodiments, the type of the failure may include a circuit disconnection, a failure of a receiving circuit, a frequency offset of the coil, or the like, or any combination thereof. In some embodiments, the processing device 140 may determine the frequency offset of the coil based on at least a portion of the set of MR signals. In some embodiments, if the FID signal decreases and the noise signal significantly increases, it may indicate that the loop of the coil is disconnected; if the FID signal decreases and the noise signal slightly increases, it may indicate that the frequency offset of the coil exists; if the FID signal disappears and the noise signals significantly decrease, it may indicate that the receiving circuit of the coil is disconnected. More descriptions of the type of the failure and/or the level of the failure may be found elsewhere in the present disclosure, for example operation 420.

In some embodiments, there may be one or more second fault detection models different from the first fault detection model. The processing device 140 determines whether the coil has a failure using the first fault detection model. In response to determining the coil has a failure, the processing device 140 determines the position of the failure using the first fault detection model and may further determine the type of the failure and/or the level of the failure using the one or more second fault detection models. Merely by way of example, if the coil has no failure, the output of the first fault detection model may be {-1}; if a channel of the coil of a serial number of 10 has a failure, the output of the first fault detection may be {10}. In response to determining the coil has no failure, the processing device 140 may set the input of the one or more second fault detection models to null.

In some embodiments, the processing device 140 may determine, based on the output of the first fault detection model, the type of the failure and/or the level of the failure using the one or more second fault detection models. The processing device 140 may determine the type and/or the level of the failure based on a portion of the one or more sets of reference signals (e.g., a portion of the set of noise signals), which is collected by the channel having the failure, using the one or more second fault detection models. For example, the processing device 140 may determine the type and/or the level of the failure by inputting the portion of the one or more sets of reference signals into the one or more second fault detection models. As another example, the processing device 140 may determine the type and/or the level of the failure by inputting information (e.g., a distribution, a probability distribution line, a feature vector) relating to the portion of the one or more sets of reference signals into the one or more second fault detection models.

In some embodiments, each of the one or more second fault detection models may be configured to detect a failure of a predetermined type. For example, the one or more second fault detection models may include a model (e.g., an SVM model) for detecting a circuit disconnection, a model (e.g., an SVM model) for detecting a failure of a receiving circuit, a model (e.g., an SVM model) for detecting a frequency offset of the coil, or the like, or any combination thereof. In some embodiments, the processing device 140 may determine the type of the failure by inputting the portion of the one or more sets of reference signals and/or the information relating to the portion of the one or more sets of reference signals, which is collected by the channel having the failure, into the one or more fault detection models, respectively. It should be noted that the descriptions herein may be non-limiting. In some embodiments, as described above, the first fault detection model may also be able to determine the type of the failure and/or the level of the failure of the coil.

In some embodiments, the one or more second fault detection models may be integrated as a single model. In some embodiments, different types of failures may be represented by first serial numbers (e.g., 1, 2, 3), that is, each type of failure may correspond to a specific first serial number. Different levels of a failure may be represented by second serial numbers (e.g., 1, 2, 3), that is, each level of failure may correspond to a specific second serial number. The processing device 140 may obtain a first relationship between the first serial numbers and the failure types and/or a second relationship between the second serial numbers and the failure levels. In some embodiments, the output of the single model may include a first serial number corresponding to the failure type and a second serial number corresponding to the failure level. For example, the output of the single model may be {1, 3}, wherein 1 is the first serial number corresponding to the failure type, and 3 is the second serial number corresponding to the failure level of the fault in the coil.

FIG. 7 is an exemplary flowchart illustrating a training process of a first fault detection model according to some embodiments of the present disclosure. The first fault detection model may be used to determine whether a coil has a failure. In some embodiments, a process 700 may be performed by a training device, such as the processing device 140, or a processing device external to the fault detection system 100 (e.g., a processing device of a vendor that generates, provides, maintains, and/or updates the first fault detection model). For illustration purposes, the implementation of the process 700 by the processing device 140 is described below. The process 700 may include following operations.

In 710, the processing device 140 (e.g., the training module 340) may obtain a plurality of first training samples and a plurality of first labels corresponding to the first training samples. The first training samples may be selected based on one or more desired functions of a trained model. For instance, the trained model may be the first fault detection model configured to determine whether a coil has a failure as described in connection with FIG. 4A (operations 420 and 430 of the process 400A).

In some embodiments, each of the first training samples may include a set of sample noise signals and a first label. The first label corresponding to the first training sample may indicate whether a sample coil has a failure. The set of sample noise signals may be collected by the sample coil in a sample acquisition that is performed on a sample subject without applying an excitation pulse to the sample subject. In some embodiments, the sample coil may be normal or abnormal. The sample coil may be the coil as described in connection with FIG. 4A and FIG. 4B, or another coil that is similar to the coil (e.g., having the same type and/or size). An image of the sample coil may be generated and used to determine whether the sample coil has a failure, a position of the failure, etc., thereby obtaining the first label corresponding to the first training sample. In some embodiments, at least a portion of a first label of a training sample may be determined manually or semi-manually. For instance, if a sample coil corresponding to a set of sample noise signals is normal, the first label may be determined as {-1} by manual annotation or other manners; or if a sample coil corresponding to a set of sample noise signals has a failure, the first label may be determined as a position of the failure (e.g., a channel number of a channel that has the failure) by manual annotation or other manners.

In 720, the processing device 140 (e.g., the training module 340) may generate the first fault detection model by training a first preliminary model based on the plurality of first training samples and the plurality of first labels. The first preliminary model may include multiple model parameters each of which is assigned with an initial value. During the training, the values of the multiple model parameters may be updated iteratively. For instance, in one iteration, at least a portion of a first training sample (e.g., all of the set of sample noise signals, a sample feature vector relating to the set of sample noise signals) may be input into the first preliminary model or an intermediate first fault detection model (that is partially training using at least one first training sample) obtained in a prior iteration (e.g., an iteration immediately preceding the current iteration), and a first prediction result may be determined. The first prediction result may indicate whether the sample coil corresponding to the set of sample noise signals has a failure. The first prediction result may be evaluated based on the first label of the first training sample.

The processing device 140 may adjust the values of model parameters of the preliminary or intermediate first fault detection model based on the evaluation of the first prediction result with the first label of the first training sample. For instance, the processing device 140 may adjust the values of model parameters of the preliminary or intermediate first fault detection model based on the evaluation of the first prediction result with the first label(s) of the first training sample to reduce the inconsistency between the first prediction result and the first label of the first training sample. Merely by way of example, the processing device 140 may obtain a first loss function that relates to the first prediction result and the first label(s) of a first training sample. The first loss function may assess whether the first prediction result and the first label are inconsistent. A value of the first loss function may be reduced or minimized (e.g., lower than a first threshold) by iteratively adjusting the values of the model parameters.

In some embodiments, as described in operation 430 above, one or more decision boundaries may be determined based on the first fault detection model. Each of the one or more decision boundaries may be used to determine a failure of a predetermined type. During the training of the first fault detection model, there may be one or more sets of first training samples corresponding to the determination of one or more decision boundaries. For example, for a decision boundary corresponding to a specific type of failure, the sample set of noise signals of each first training sample may be collected by a sample coil having the specific type of failure. Accordingly, after the training, the first fault detection model may be able to detect specific types of failure based on the one or more determined decision boundaries.

FIG. 8 is an exemplary flowchart illustrating a training process of a second fault detection model according to some embodiments of the present disclosure. The second fault detection model may be used to determine additional information (e.g., a type of a failure, a level of a failure) of a failure. In some embodiments, a process 800 may be performed by a training device, such as the processing device140, a different processing device of the fault detection system 100, or a processing device external to the fault detection system 100 (e.g., a processing device of a vendor that generates, provides, maintains, and/or updates the one or more second fault detection model). For illustration purposes, the implementation of the process 800 by the processing device 140 is described below. The process 800 may include following operations.

In 810, the processing device 140 (e.g., the training module 340) may obtain a plurality of second training samples and a plurality of second labels corresponding to the second training samples. The second training samples may be selected based on one or more desired functions of a trained model. For instance, the trained model may be a second fault detection model configured to determine additional information (e.g., a type of a failure, a level of a failure) of a failure as illustrated in FIG. 4A (operations 420 and 440 of the process 400A).

In some embodiments, each of the second training samples may include a set of sample noise signals and/or a set of sample MR signals and a second label. The set of sample noise signals and/or the set of sample MR signals may be collected by an abnormal sample coil (that is, the sample coil has a failure). The set of sample noise signals may be collected by the abnormal sample coil in a sample acquisition that is performed on a sample subject without applying an excitation pulse to the sample subject. The set of sample MR signals may be collected in a sample acquisition that is performed on the sample subject after an excitation pulse is applied to the sample subject. The second label corresponding to the second training sample may include additional information of a failure. The additional information of the failure may include a type of the failure and/or a level of the failure. An image of the abnormal sample coil may be generated and used to determine the type of the failure, the level of the failure, etc., thereby obtaining the second label corresponding to the second training sample. In some embodiments, at least a portion of a second label of a training sample may be determined manually or semi-manually.

In 820, the processing device 140 (e.g., the training module 340) may generate a second fault detection model by training a second preliminary model based on the plurality of second training samples and the plurality of second labels. The second preliminary model may include multiple model parameters each of which is assigned with an initial value. During the training, the values of the multiple model parameters may be updated iteratively. For instance, in one iteration, at least a portion of a second training sample (e.g., the set of sample noise signals, the set of sample MR signals, a sample feature vector relating to the set of sample noise signals) may be input into the second preliminary model or an intermediate second fault detection model (that is partially training using at least one second training sample) obtained in a prior iteration (e.g., an iteration immediately preceding the current iteration) and a second prediction result may be determined. The second prediction result may include predicted additional information of a failure, such as the type of the failure and/or the level of the failure. The second prediction result may be evaluated based on the second label(s) of the second training sample.

The processing device 140 may adjust the values of model parameters of the preliminary or intermediate second fault detection model based on the evaluation of the second prediction result with the second label(s) of the second training sample. For instance, the processing device 140 may adjust the values of model parameters of the preliminary or intermediate second fault detection model based on the evaluation of the second prediction result with the second label(s) of the second training sample to reduce the inconsistency between the second prediction result and the second label(s) of the second training sample. Merely by way of example, the processing device 140 may obtain a second loss function that relates to the second prediction result and the second label(s) of a second training sample. The second loss function may assess whether the second prediction result and the second label are inconsistent. A value of the second loss function may be reduced or minimized (e.g., lower than a second threshold) by iteratively adjusting the values of the model parameters.

In some embodiments, as described in operations 420 and 440, each of the one or more second fault detection models may be configured to determine a failure of a predetermined type. There may be one or more sets of second training samples corresponding to the one or more second fault detection models. For example, for a second fault detection model corresponding to a specific type of failure, the set of sample noise signals and/or the set of sample MR signals of each first training sample, which are collected by a sample coil having the specific type of failure, may be used to train the second fault detection model. Accordingly, the one or more second fault detection models may be generated by training one or more second preliminary models based on one or more sets of second training samples, respectively.

In some embodiments, the functions of the first fault detection model and the one or more second fault detection models may be achieved by a single model. It should be noted that the single model may be trained similar to the process 700 and/or the process 800 using suitable training samples.

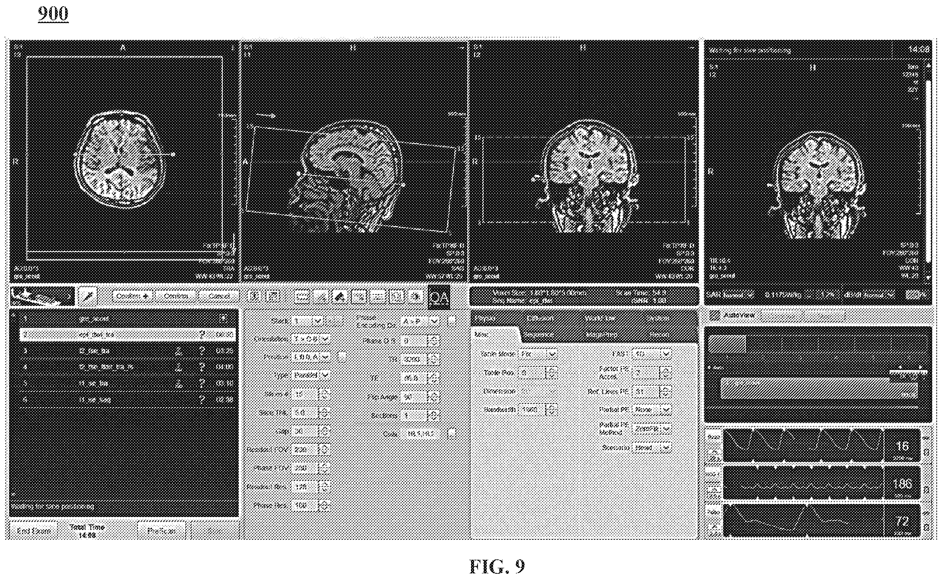

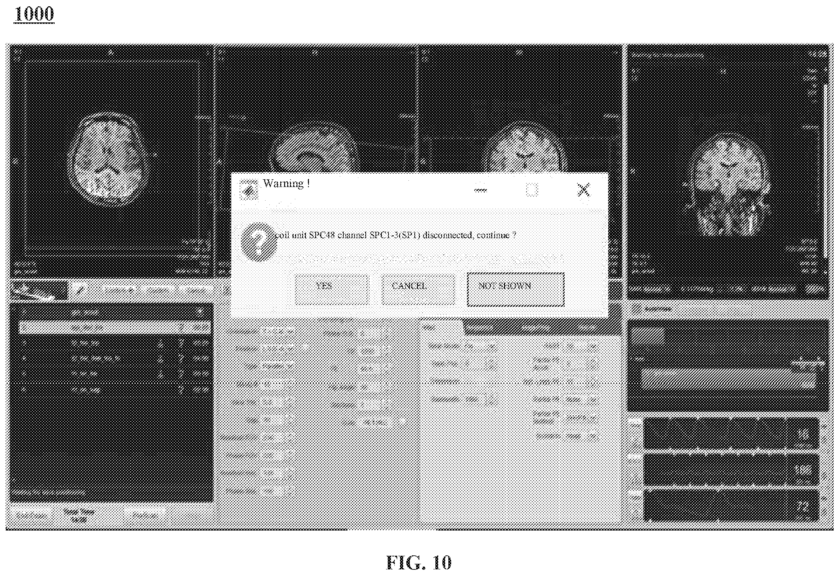

FIG. 9 is a schematic diagram illustrating an exemplary interface of a terminal according to some embodiments of the present disclosure. FIG. 10 is a schematic diagram illustrating another exemplary interface of a terminal according to some embodiments of the present disclosure. In some embodiments, the terminal may include the processing device 140.

As shown in FIG. 9, an interface 900 of the terminal may include a quality assurance (QA) button. In some scenarios (e.g., in a clinical scenario), the coil failure detection function may be turned on or off through the QA button. By turning on the QA button, the coil failure detection function may be initiated. In response to determining that a coil has a failure, a warning dialog box 1000 may pop up as shown in FIG. 10. In some embodiments, the fault detection result of the coil may be recorded in a log folder, facilitating an operator (e.g., an engineer) to debug the coil failure. In some scenarios (e.g., in a scientific research scenario), the QA button may be set to be initiated by default. In response to determining the coil has a failure, a warning dialog box 1000 may pop up. An operator may choose whether to continue a scanning. In some embodiments, the interface 900 of the terminal may also display a toolbox for viewing the fault detection result of the coil, that is, the toolbox may store the fault detection result of the coil.

In some embodiments, when a condition is satisfied, the coil failure detection function may be initiated. The condition may include a sag or deflection of the couch, re-plugging the coil, re-selection of the coil, or the like, or any combination thereof. For example, the QA procedure may compare a name of a current coil with a name of a historical coil used in the MRI device. In response to determining that the name of the current coil and the name of the historical coil are inconsistent, the QA procedure may be initiated; in response to determining the name of the current coil and the name of the historical coil are consistent, the QA procedure may be skipped.

In some embodiments, when the QA procedure is initiated, the terminal may search for one or more sets of reference signals and perform the failure detection function based on the set(s) of reference signals (or a portion thereof). The terminal may also record a log to mark the detection time. In response to determining the coil has a failure, a warning window may pop up to remind an operator whether to stop the scanning.

In some embodiments, the process for coil fault detection may be illustrated below. The QA button may be initiated. In response to determining that the coil has a failure, a warning dialog box 1000 may pop up, and whether to continue a scanning may be prompted. By clicking "OK," the scanning may continue; by clicking "Cancel", the scanning may be stopped; by clicking "Don't Display," the scanning may continue, and no more reminders may pop up before the scanning ends. The interface 900 may also include an adaptive toolbox storing the fault detection result, and an authorized operator (e.g., a scientific user) may view the fault detection result in the adaptive toolbox.

In some embodiments, the process for coil fault detection may be illustrated below. The QA button may be initiated. In response to determining that the coil has a failure, a warning may appear in the interface 900 to prompt whether to continue a scanning.

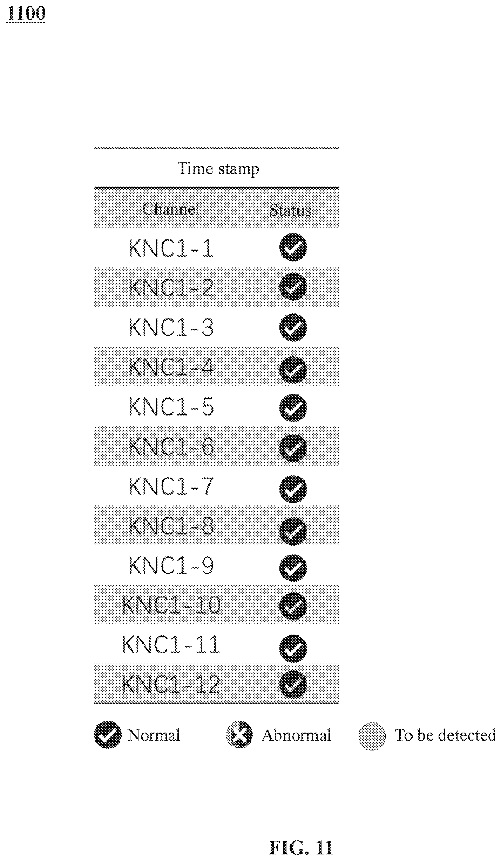

FIG. 11 is a schematic diagram illustrating an exemplary log according to some embodiments of the present disclosure. The log may be used to store a fault detection result of a coil.

As shown in FIG. 11, the log may include a timestamp, a type of a coil, serial numbers of different channels of the coil, a status of each channel of the coil, etc. The timestamp may represent the time of failure detection of the coil. "KNC1" may indicate the type of the coil. The digit after the type of the coil may indicate a serial number of a channel of the coil. The status of the coil may include a normal status, an abnormal status, a to-be-detected status, etc.

## Claims

1. A system (100), comprising:
at least one storage device (150) including a set of instructions;
at least one processor (140) in communication with the at least one storage device (150), wherein when executing the set of instructions, the at least one processor (140) is configured to cause the system (100) to perform operations including:
obtaining (410) one or more sets of reference signals collected by a coil of a magnetic resonance imaging (MRI) device (110) in a pre-scan, the pre-scan being performed on a subject before an MRI scan of the subject, wherein the coil includes multiple channels, each of the multiple channels is configured to collect a portion of the one or more sets of reference signals;
obtaining (420) a first fault detection model, the first fault detection model being a trained machine learning model; and
determining (430) whether the coil has a failure by inputting the one or more sets of reference signals into the first fault detection model, wherein an output of the first fault detection model includes a determination result of whether the coil has a failure, and whether the coil has a failure is determined based on the determination result; and
in response to determining the coil has a failure, determining (440) a position of the failure of the coil based on the output of the first fault detection model, wherein the position of the failure includes a serial number of a channel that has the failure among the plurality of channels, and the output further includes the serial number when the coil has a failure.

2. The system of claim 1, wherein the one or more sets of reference signals include a set of MR signals collected in an acquisition that is performed on the subject after an excitation pulse is applied to the subject.

3. The system of claim 1 or claim 2, wherein the one or more sets of reference signals include a set of noise signals collected in an acquisition that is performed on the subject without applying an excitation pulse to the subject.

4. The system of any one of the preceding claims, the operations further comprising:
in response to determining the coil has a failure, determining a type or a level of the failure using one or more second fault detection models different from the first fault detection model.

5. The system of claim 4, wherein the type of the failure includes at least one of
a circuit disconnection,
a failure of a receiving circuit, or
a frequency offset of the coil.

6. The system of any one of the preceding claims, the operations further comprising:
obtaining device information of the MRI device (110); and
determining whether the coil has a failure by inputting the one or more sets of reference signals and the device information into the first fault detection model.

7. A method implemented on a computing device (200) having at least one processor (150) and at least one storage device (150), comprising:
obtaining (410) one or more sets of reference signals collected by a coil of a magnetic resonance imaging (MRI) device (110) in a pre-scan, the pre-scan being performed on a subject before an MRI scan of the subject, wherein the coil includes multiple channels, each of the multiple channels is configured to collect a portion of the one or more sets of reference signals;
obtaining (420) a first fault detection model, the first fault detection model being a trained machine learning model; and
determining (430) whether the coil has a failure by inputting the one or more sets of reference signals into the first fault detection model, wherein an output of the first fault detection model includes a determination result of whether the coil has a failure, and whether the coil has a failure is determined based on the determination result; and
in response to determining the coil has a failure, determining (440) a position of the failure of the coil based on the output of the first fault detection model, wherein the position of the failure includes a serial number of a channel that has the failure among the plurality of channels, and the output further includes the serial number when the coil has a failure.

8. The method of claim 7, wherein the one or more sets of reference signals include a set of MR signals collected in an acquisition that is performed on the subject after an excitation pulse is applied to the subject.

9. The method of claim 7 or claim 8, wherein the one or more sets of reference signals include a set of noise signals collected in an acquisition that is performed on the subject without applying an excitation pulse to the subject.

10. A non-transitory computer readable medium including executable instructions, the instructions, when executed by at least one processor (140), causing the at least one processor (140) to effectuate a method comprising:
obtaining (410) one or more sets of reference signals collected by a coil of a magnetic resonance imaging (MRI) device (110) in a pre-scan, the pre-scan being performed on a subject before an MRI scan of the subject, wherein the coil includes multiple channels, each of the multiple channels is configured to collect a portion of the one or more sets of reference signals,
obtaining (420) a first fault detection model, the first fault detection model being a trained machine learning model; and
determining (430) whether the coil has a failure by inputting the one or more sets of reference signals into the first fault detection model, wherein an output of the first fault detection model includes a determination result of whether the coil has a failure, and whether the coil has a failure is determined based on the determination result; and
in response to determining the coil has a failure, determining (440) a position of the failure of the coil based on the output of the first fault detection model, wherein the position of the failure includes a serial number of a channel that has the failure among the plurality of channels, and the output further includes the serial number when the coil has a failure.

## Patentansprüche

1. System (100), umfassend:
mindestens eine Speichervorrichtung (150), die einen Satz von Anweisungen beinhaltet;
mindestens einen Prozessor (140), in Kommunikation mit der mindestens einen Speichervorrichtung (150), wobei der mindestens eine Prozessor (140), wenn er den Satz von Anweisungen ausführt, dazu konfiguriert ist, das System (100) zu veranlassen, Operationen durchzuführen, die Folgendes beinhalten:
Erhalten (410) eines oder mehrerer Sätze von Referenzsignalen, die von einer Spule einer Magnetresonanztomographie- (MRT) Vorrichtung (110) in einem Vorscan erhoben werden, wobei der Vorscan an einem Subjekt vor einem MRT-Scan des Subjekts durchgeführt wird, wobei die Spule mehrere Kanäle beinhaltet und jeder der mehreren Kanäle dazu konfiguriert ist, einen Teil des einen oder der mehreren Sätze von Referenzsignalen zu erheben;
Erhalten (420) eines ersten Fehlererkennungsmodells, wobei das erste Fehlererkennungsmodell ein trainiertes Maschinenlernmodell ist; und
Bestimmen (430), ob die Spule einen Ausfall aufweist, indem der eine oder die mehreren Sätze von Referenzsignalen in das erste Fehlererkennungsmodell eingegeben werden, wobei eine Ausgabe des ersten Fehlererkennungsmodells ein Bestimmungsergebnis darüber beinhaltet, ob die Spule einen Ausfall aufweist, und ob die Spule einen Ausfall aufweist, basierend auf dem Bestimmungsergebnis bestimmt wird; und
als Reaktion auf Bestimmen, dass die Spule einen Ausfall aufweist, Bestimmen (440) einer Position des Ausfalls der Spule basierend auf der Ausgabe des ersten Fehlererkennungsmodells, wobei die Position des Ausfalls eine Seriennummer eines Kanals beinhaltet, der unter der Vielzahl von Kanälen den Ausfall aufweist, und die Ausgabe weiter die Seriennummer beinhaltet, wenn die Spule einen Ausfall aufweist.

2. System nach Anspruch 1, wobei der eine oder die mehreren Sätze von Referenzsignalen einen Satz von MR-Signalen beinhalten, die bei einer Erfassung erhoben werden, die an dem Subjekt durchgeführt wird, nachdem ein Anregungsimpuls auf das Subjekt angewendet wurde.

3. System nach Anspruch 1 oder Anspruch 2, wobei der eine oder die mehreren Sätze von Referenzsignalen einen Satz von Rauschsignalen beinhalten, die bei einer Erfassung erhoben werden, die an dem Subjekt durchgeführt wird, ohne ein Anregungsimpuls auf das Subjekt anzuwenden.

4. System nach einem der vorstehenden Ansprüche, wobei die Operationen weiter Folgendes umfassen:
als Reaktion auf Bestimmen, dass die Spule einen Ausfall aufweist, Bestimmen eines Typs oder eines Ausmaßes des Ausfalls unter Verwendung eines oder mehrerer zweiter Fehlererkennungsmodelle, die sich vom ersten Fehlererkennungsmodell unterscheiden.

5. System nach Anspruch 4, wobei der Typ des Ausfalls mindestens eines umfasst von einer Schaltungsunterbrechung,
einem Ausfall einer Empfangsschaltung oder
einem Frequenzoffset der Spule.

6. System nach einem der vorstehenden Ansprüche, wobei die Operationen weiter Folgendes umfassen:
Erhalten von Vorrichtungsinformationen der MRT-Vorrichtung (110); und
Bestimmen, ob die Spule einen Ausfall aufweist, indem der eine oder die mehreren Sätze von Referenzsignalen und die Vorrichtungsinformationen in das erste Fehlererkennungsmodell eingegeben werden.

7. Verfahren, das auf einer Rechenvorrichtung (200) implementiert wird, die mindestens einen Prozessor (150) und mindestens eine Speichervorrichtung (150) aufweist, wobei es Folgendes umfasst:
Erhalten (410) eines oder mehrerer Sätze von Referenzsignalen, die von einer Spule einer Magnetresonanztomographie- (MRT) Vorrichtung (110) in einem Vorscan erhoben werden, wobei der Vorscan an einem Subjekt vor einem MRT-Scan des Subjekts durchgeführt wird, wobei die Spule mehrere Kanäle beinhaltet und jeder der mehreren Kanäle dazu konfiguriert ist, einen Teil des einen oder der mehreren Sätze von Referenzsignalen zu erheben;
Erhalten (420) eines ersten Fehlererkennungsmodells, wobei das erste Fehlererkennungsmodell ein trainiertes Maschinenlernmodell ist; und
Bestimmen (430), ob die Spule einen Ausfall aufweist, indem der eine oder die mehreren Sätze von Referenzsignalen in das erste Fehlererkennungsmodell eingegeben werden, wobei eine Ausgabe des ersten Fehlererkennungsmodells ein Bestimmungsergebnis darüber beinhaltet, ob die Spule einen Ausfall aufweist, und ob die Spule einen Ausfall aufweist, basierend auf dem Bestimmungsergebnis bestimmt wird; und
als Reaktion auf Bestimmen, dass die Spule einen Ausfall aufweist, Bestimmen (440) einer Position des Ausfalls der Spule basierend auf der Ausgabe des ersten Fehlererkennungsmodells, wobei die Position des Ausfalls eine Seriennummer eines Kanals beinhaltet, der unter der Vielzahl von Kanälen den Ausfall aufweist, und die Ausgabe weiter die Seriennummer beinhaltet, wenn die Spule einen Ausfall aufweist.

8. Verfahren nach Anspruch 7, wobei der eine oder die mehreren Sätze von Referenzsignalen einen Satz von MR-Signalen beinhalten, die bei einer Erfassung erhoben werden, die an dem Subjekt durchgeführt wird, nachdem ein Anregungsimpuls auf das Subjekt angewendet wurde.

9. Verfahren nach Anspruch 7 oder Anspruch 8, wobei der eine oder die mehreren Sätze von Referenzsignalen einen Satz von Rauschsignalen beinhalten, die bei einer Erfassung erhoben werden, die an dem Subjekt durchgeführt wird, ohne ein Anregungsimpuls auf das Subjekt anzuwenden.

10. Nichtflüchtiges computerlesbares Medium, das ausführbare Anweisungen beinhaltet, wobei die Anweisungen, wenn sie von mindestens einem Prozessor (140) ausgeführt werden, den mindestens einen Prozessor (140) dazu veranlassen, ein Verfahren umzusetzen, das Folgendes umfasst:
Erhalten (410) eines oder mehrerer Sätze von Referenzsignalen, die von einer Spule einer Magnetresonanztomographie- (MRT) Vorrichtung (110) in einem Vorscan erhoben werden, wobei der Vorscan an einem Subjekt vor einem MRT-Scan des Subjekts durchgeführt wird, wobei die Spule mehrere Kanäle beinhaltet und jeder der mehreren Kanäle dazu konfiguriert ist, einen Teil des einen oder der mehreren Sätze von Referenzsignalen zu erheben;
Erhalten (420) eines ersten Fehlererkennungsmodells, wobei das erste Fehlererkennungsmodell ein trainiertes Maschinenlernmodell ist; und
Bestimmen (430), ob die Spule einen Ausfall aufweist, indem der eine oder die mehreren Sätze von Referenzsignalen in das erste Fehlererkennungsmodell eingegeben werden, wobei eine Ausgabe des ersten Fehlererkennungsmodells ein Bestimmungsergebnis darüber beinhaltet, ob die Spule einen Ausfall aufweist, und ob die Spule einen Ausfall aufweist, basierend auf dem Bestimmungsergebnis bestimmt wird; und
als Reaktion auf Bestimmen, dass die Spule einen Ausfall aufweist, Bestimmen (440) einer Position des Ausfalls der Spule basierend auf der Ausgabe des ersten Fehlererkennungsmodells, wobei die Position des Ausfalls eine Seriennummer eines Kanals beinhaltet, der unter der Vielzahl von Kanälen den Ausfall aufweist, und die Ausgabe weiter die Seriennummer beinhaltet, wenn die Spule einen Ausfall aufweist.

## Revendications

1. Système (100), comprenant :
au moins un dispositif de stockage (150) incluant un ensemble d'instructions ;
au moins un processeur (140) en communication avec le au moins un dispositif de stockage (150), dans lequel, lors de l'exécution de l'ensemble d'instructions, le au moins un processeur (140) est configuré pour amener le système à effectuer des opérations incluant :
obtenir (410) un ou plusieurs ensembles de signaux de référence collectés par une bobine d'un dispositif d'imagerie par résonance magnétique (IRM) (110) dans un pré-balayage, le pré-balayage étant effectué sur un sujet avant un balayage d'IRM du sujet, dans lequel la bobine inclut de multiples canaux, chacun des multiples canaux est configuré pour collecter une partie des un ou plusieurs ensembles de signaux de référence ;
obtenir (420) un premier modèle de détection de défaut, le premier modèle de détection de défaut étant un modèle d'apprentissage automatique entraîné ; et
déterminer (430) si la bobine présente une défaillance en entrant les un ou plusieurs ensembles de signaux de référence dans le premier modèle de détection de défaut, dans lequel une sortie du premier modèle de détection de défaut inclut un résultat de détermination de la présence ou non d'une défaillance de la bobine, et la présence ou non d'une défaillance de la bobine est déterminée sur la base du résultat de détermination ; et
en réponse à la détermination de la présence d'une défaillance de la bobine, déterminer (440) une position de la défaillance de la bobine sur la base de la sortie du premier modèle de détection de défaut, dans lequel la position de la défaillance inclut un numéro de série d'un canal qui présente la défaillance parmi la pluralité de canaux, et la sortie inclut en outre le numéro de série lorsque la bobine présente une défaillance.

2. Système selon la revendication 1, dans lequel les un ou plusieurs ensembles de signaux de référence incluent un ensemble de signaux RM collectés dans une acquisition qui est effectuée sur le sujet après qu'une impulsion d'excitation a été appliquée au sujet.

3. Système selon la revendication 1 ou la revendication 2, dans lequel les un ou plusieurs ensembles de signaux de référence incluent un ensemble de signaux de bruit collectés dans une acquisition qui est effectuée sur le sujet sans appliquer une impulsion d'excitation au sujet.

4. Système selon l'une quelconque des revendications précédentes, les opérations comprenant en outre :
en réponse à la détermination de la présence d'une défaillance de la bobine, déterminer un type ou un niveau de la défaillance à l'aide d'un ou de plusieurs seconds modèles de détection de défaut différents du premier modèle de détection de défaut.

5. Système selon la revendication 4, dans lequel le type de la défaillance inclut au moins un parmi :
une déconnexion de circuit,
une défaillance d'un circuit de réception, ou
un décalage de fréquence de la bobine.

6. Système selon l'une quelconque des revendications précédentes, les opérations comprenant en outre :
obtenir des informations de dispositif du dispositif d'IRM (110) ; et
déterminer si la bobine présente une défaillance en entrant les un ou plusieurs ensembles de signaux de référence et les informations de dispositif dans le premier modèle de détection de défaut.

7. Procédé mis en œuvre sur un dispositif informatique (200) présentant au moins un processeur (150) et au moins un dispositif de stockage (150), comprenant :
l'obtention (410) d'un ou de plusieurs ensembles de signaux de référence collectés par une bobine d'un dispositif d'imagerie par résonance magnétique (IRM) (110) dans un pré-balayage, le pré-balayage étant effectué sur un sujet avant un balayage d'IRM du sujet, dans lequel la bobine inclut de multiples canaux, chacun des multiples canaux est configuré pour collecter une partie des un ou plusieurs ensembles de signaux de référence ;
l'obtention (420) d'un premier modèle de détection de défaut, le premier modèle de détection de défaut étant un modèle d'apprentissage automatique entraîné ; et
la détermination (430) de la présence ou non d'une défaillance de la bobine en entrant les un ou plusieurs ensembles de signaux de référence dans le premier modèle de détection de défaut, dans lequel une sortie du premier modèle de détection de défaut inclut un résultat de détermination de la présence ou non d'une défaillance de la bobine, et la présence ou non d'une défaillance de la bobine est déterminée sur la base du résultat de détermination ;
et
en réponse à la détermination de la présence d'une défaillance de la bobine, la détermination (440) d'une position de la défaillance de la bobine sur la base de la sortie du premier modèle de détection de défaut, dans lequel la position de la défaillance inclut un numéro de série d'un canal qui présente la défaillance parmi la pluralité de canaux, et la sortie inclut en outre le numéro de série lorsque la bobine présente une défaillance.

8. Procédé selon la revendication 7, dans lequel les un ou plusieurs ensembles de signaux de référence incluent un ensemble de signaux RM collectés dans une acquisition qui est effectuée sur le sujet après qu'une impulsion d'excitation a été appliquée au sujet.

9. Procédé selon la revendication 7 ou la revendication 8, dans lequel les un ou plusieurs ensembles de signaux de référence incluent un ensemble de signaux de bruit collectés dans une acquisition qui est effectuée sur le sujet sans appliquer une impulsion d'excitation au sujet.

10. Support non transitoire lisible par ordinateur incluant des instructions exécutables qui, lorsqu'elles sont exécutées par au moins un processeur (140), amènent le au moins un processeur (140) à effectuer un procédé comprenant :
l'obtention (410) d'un ou de plusieurs ensembles de signaux de référence collectés par une bobine d'un dispositif d'imagerie par résonance magnétique (IRM) (110) dans un pré-balayage, le pré-balayage étant effectué sur un sujet avant un balayage d'IRM du sujet, dans lequel la bobine inclut de multiples canaux, chacun des multiples canaux est configuré pour collecter une partie des un ou plusieurs ensembles de signaux de référence ;
l'obtention (420) d'un premier modèle de détection de défaut, le premier modèle de détection de défaut étant un modèle d'apprentissage automatique entraîné ; et
la détermination (430) de la présence ou non d'une défaillance de la bobine en entrant les un ou plusieurs ensembles de signaux de référence dans le premier modèle de détection de défaut, dans lequel une sortie du premier modèle de détection de défaut inclut un résultat de détermination de la présence ou non d'une défaillance de la bobine, et la présence ou non d'une défaillance de la bobine est déterminée sur la base du résultat de détermination ; et
en réponse à la détermination de la présence d'une défaillance de la bobine, la détermination (440) d'une position de la défaillance de la bobine sur la base de la sortie du premier modèle de détection de défaut, dans lequel la position de la défaillance inclut un numéro de série d'un canal qui présente la défaillance parmi la pluralité de canaux, et la sortie inclut en outre le numéro de série lorsque la bobine présente une défaillance.
